Europäisches Patentamt

European Patent Office

Office européen des brevets

(19)

(11) Numéro de publication: **0 095 950
B1**

(12) # FASCICULE DE BREVET EUROPEEN

(45) Date de publication du fascicule du brevet:
08.10.86

(51) Int. Cl.⁴: **C 12 P 13/00, C 12 N 9/04**

(21) Numéro de dépôt: 83400758.5

(22) Date de dépôt: 18.04.83

(54) **Production de glucose deshydrogenase.**

(30) Priorité: 06.05.82 FR 8208036

(43) Date de publication de la demande:
07.12.83 Bulletin 83/49

(45) Mention de la délivrance du brevet:
08.10.86 Bulletin 86/41

(84) Etats contractants désignés:
AT BE CH DE GB IT LI LU NL SE

(56) Documents cités:
FR - A - 2 398 046

CHEMICAL ABSTRACTS, vol. 86, no. 11, 14 mars 1977,
page 257, no. 68060a, Columbus, Ohio, US, S.
CHATELAIN et al.: "Changes in enzyme activities in an
asporogenous mutant of Bacillus megaterium in various
nutritionally deficient media"
CHEMICAL ABSTRACTS, vol. 92, no. 21, 26 mai 1980,
page 477, no. 179032m, Columbus, Ohio, US,
VANDECASTEELE et al.: "Reductive enzymic synthesis
of L-carnitine with regeneration of the NADH used"
CHEMICAL ABSTRACTS, vol. 92, no. 19, 12 mai 1980,
page 233, no. 159667u, Columbus, Ohio, US,
VANDECASTEELE: "Enzymic synthesis of L-carnitine
by reduction of an achiral precursor: the problem of
reduced nicotinamide adenine dinucleotide recycling"

(73) Titulaire: INSTITUT FRANCAIS DU PETROLE, 4, Avenue
de Bois-Préau, F-92502 Rueil-Malmaison (FR)

(72) Inventeur: Vandecasteele, Jean-Paul, 1, Allée des
Ormes, F-78112 Fourqueux (FR)
Inventeur: Ballerini, Daniel, 103, rue de Pontel,
F-78100 Saint Germain en Laye (FR)
Inventeur: Lemal, Jeanine, 3, Chemin de Paradis,
F-92500 Rueil-Malmaison (FR)
Inventeur: Le Penru, Yann, 8, rue André Derain,
F-78400 Chatou (FR)

(56) Documents cités: (suite)
CHEMICAL ABSTRACTS, vol. 84, no. 7, 16 février 1976,
page 212, no. 40643C, Columbus, Ohio, US, S.
CHATELAIN: "Changes in enzyme activities under
different conditions of sporulation of Bacillus
megaterium"

## Description

Un procédé de préparation de la L-carnitine par réduction asymétrique de la 3-déshydrocarnitine a été décrit dans le brevet français N° 2 398 046. Le procédé consiste à soumettre la 3-déshydrocarnitine, dont la synthèse chimique a été décrite par exemple par Aurich et coll., Hoppe-Seyler's Z. Physiol. Chem. 349 1310 (1968), ou un de ses sels, à l'action simultanée en milieu aqueux des éléments suivants:

a) La carnitine déshydrogénase

b) Un coenzyme utilisable par la carnitine déshydrogénase pour la réduction de la déshydrocarnitine, ce coenzyme étant le nicotinamide adénine dinucléotide, lequel peut exister sous forme oxydée (NAD$^+$) ou sous forme réduite (NADH).

c) Un système (ou agent) chimique ou enzymatique de réduction de la forme oxydée du nicotinamide adénine dinucléotide. Ce système comporte dans tous les cas un réducteur (R) et, en outre, dans le cas d'un système de réduction enzymatique un enzyme (E).

Les conditions de mise en œuvre de la réaction de préparation de la L-carnitine sont décrites dans le brevet français précité. Divers systèmes de réduction du NAD+ (encore appelés systèmes de régénération du NADH) ont été décrits dans le brevet français déjà cité notamment des systèmes enzymatiques (composés alors d'un réducteur R et d'un enzyme E). Les résultats les meilleurs ont été obtenus avec un système où le réducteur est le glucose et l'enzyme la glucose déshydrogénase (E.C. 1.1.1.47).

La réaction de réduction du NAD+ s'écrit alors:

$$\text{Glucose} + \text{NAD} + \xrightarrow[\text{(E)}]{\text{glucose déshydrogénase}} \text{gluconolactone} + \text{NADH} + \text{H}^+$$
$$\text{(R)}$$

Cependant les bonnes performances obtenues en utilisant ce système avaient nécessité l'emploi d'une glucose déshydrogénase purifiée. Ces conditions ne sont pas utilisables industriellement, le coût de la purification d'un enzyme, en l'occurence la glucose déshydrogénase, étant généralement très élevé. L'emploi d'un extrait brut (c'est à dire de la préparation obtenue après cassage d'une suspension de microorganismes contenant l'activité enzymatique recherchée et élimination, par exemple par centrifugation, des débris de cellules microbiennes) ou d'un extrait très peu purifié contenant l'activité enzymatique de la glucose déshydrogénase est donc susceptible d'abaisser considérablement ce coût. Malheureusement, dans de nombreux cas, cet emploi s'avère impossible du fait de la trop faible activité des extraits bruts qui exige la mise en œuvre de trop grandes quantités d'extraits bruts ainsi que du fait de la présence dans ces extraits de composés divers, qui peuvent être eux-mêmes des enzymes, et qui exercent d'une façon ou d'une autre une action défavorable sur la synthèse considérée.

Les origines possibles de cette action défavorable sont diverses et parfois cumulatives et ne sont pas toujours connues. On peut citer par exemple l'inactivation trop rapide des enzymes et coenzymes utilisés dans la réaction par suite de la présence de l'extrait brut, la synthèse de produits différents à partir des substrats présents et l'inhi-bition de la synthèse de la L-carnitine par les produits contenus dans l'extrait. Un autre inconvénient de l'emploi d'extraits enzymatiques bruts réside dans la difficulté de purification de la L-carnitine par suite de la présence des impuretés contenues dans l'extrait brut utilisé.

Le demandeur a découvert les conditions permettant la préparation d'un extrait brut stable et actif de glucose déshydrogénase et son utilisation pour la synthèse de L-carnitine.

Les expériences qui ont conduit à la découverte du procédé de l'invention sont résumées ci-après:

L'activité de la glucose déshydrogénase a été mesurée dans des extraits enzymatiques provenant de diverses souches de bactéries appartenant à l'espèce Bacillus subtilis. On a constaté que certains mutants de sporulation (mutants formant, à la fin de la croissance, des spores incomplètement dévelopées) avaient une activité glucose déshydrogénase beaucoup plus élevée que celle de la souche sauvage. Des résultats analogues ont été observés avec des souches appartenant à d'autres espèces de Bacillus telles que Bacillus cereus. Dans de nombreux cas cependant, la stabilité des extraits enzymatiques était mauvaise, la glucose déshydrogénase perdant une grande partie de son activité en quelques heures, alors que la stabilité du même enzyme après purification était beaucoup meilleure. La raison de cette instabilité n'a pas été élucidée mais il est possible qu'elle soit dûe au moins en partie à la présence de protéases qui inactivent les enzymes tels que la glucose déshydrogénase en hydrolysant certaines des liaisons peptidiques de ces enzymes. On a maintenant découvert que, chez un mutant de sporulation particulier de Bacillus megaterium, la glucose déshydrogénase dans les extraits bruts a une activité élevée et une stabilité meilleure que dans le cas précédent. Ce mutant a été obtenu à partir de la souche de la collection du laboratoire, Bacillus megaterium IFP 180. Cette souche est une bactérie aérobie en forme de bâtonnets formant des endospores résistantes à la température dont les caractéristique correspondent à l'espèce Bacillus megaterium telle que décrite dans le «Bergey's manual of determinative bacteriology» 8ème édition (1974), The Williams & Wilkins Company, Balti-more, p. 529. On a effectué des repiquages successifs sur des milieux glucosés neufs, de cultures de B. megaterium IFP 180 ayant terminé leur croissance et effectué leur sporulation. On a constaté que l'activité glucose déshydrogénase des extraits enzymatiques obtenus à partir des cultures ayant subi plusieurs repiquages successifs était considérablement augmentée. Ainsi, par exemple, après douze repiquages successifs, on a purifié la culture par isolement d'une colonie après étalement sur milieu solide. La nouvelle souche obtenue, dénommée Bacillus megaterium IFP 18, présente des caractères semblables à ceux de la souche mère B. megaterium IFP 180 mais s'en distingue par un taux de sporulation becaucoup plus faible et une activité glucose déshydrogénase nettement plus élevée. On en a conclu que les repiquages effectués, qui sont connus pour favoriser la sélection de mutants asporogènes [J.P. Aubert, J. Millet, E. Pineau et G. Milhaud, Biochim. Biophys. Acta, 51, (1961) 529–537] avaient effectivement conduit à l'obtention d'un tel mutant qui présente par ailleurs la propriété de posséder une activité glucose déshydrogénase élevée. La souche IFP 188 porte le numéro ATCC 39.118.

Le procédé de l'invention consiste donc à produire de la glucose déshydrogénase par culture du mutant IFP 188 (ATCC 39.118) de Bacillus megaterium, obtenu par repiquages successifs sur des milieux glucosés d'une souche de Bacillus megaterium ayant terminé sa croissance et effectué sa sporulation.

On a également observé que la composition du milieu nutritif employé pour la culture des souches de Bacillus a une influence considérable sur l'activité glucose déshydrogénase des préparations enzymatiques obtenues à partir de ces cultures. La présence de glucose dans le milieu à une concentration comprise entre 1 et 40 g/l s'est avérée indispensable pour l'obtention d'une bonne activité glucose déshydrogénase. D'une façon générale l'addition d'une source d'aminoacides telle que peptone de soja, hydrolysats de caséine, liqueur de macération de maïs («corn steep liquor»), extrait de viande, par exemple, est avantageusement pratiquée pour l'obtention d'une bonne croissance et d'une activité enzymatique élevée.

Contrairement au mutant Bacillus megaterium KM 59, décrit par Chatelain et Fargette, C.R. Hebd. Seances Acad. Sci., série D 1976, 283 (13), pages 1563–1566, qui ne produit de la glucose déshydrogénase que dans des conditions de pénurie en phosphate, la souche IFP 188 possède une activité glucose déshydrogénase importante même lorsqu'elle est cultivée en présence d'un excès de phosphate.

L'addition de glucose dans le milieu peut être effectuée en une seule fois au début de la culture. Dans certains cas, des activités enzymatiques plus élevées ont été obtenues par addition progressive de glucose au cours de la fermentation.

La proportion de glucose, dans la culture, est de préférence de 1 à 40 g/l. Un milieu préféré renferme au moins 1 g/l de glucose et au moins 1 g/l de liqueur de trempe de maïs.

A partir des cultures de Bacillus megaterium IFP 188, on prépare des extraits enzymatiques bruts ayant une activité glucose déshydrogénase élevée en récoltant les bactéries par centrifugation puis en cassant des suspensions de ces bactéries dans un tampon par les techniques connues telles que le traitement avec les ultrasons, le broyage avec des billes de verre etc. Après centrifugation de suspension bactériennes cassées pour éliminer les débris cellulaires, on obtient les extraits enzymatiques bruts de glucose déshydrogénase utilisés pour la synthèse de la L-carnitine.

On a étudié la stabilité de la glucose déshydrogénase dans les extraits bruts obtenus à partir de la souche IFP 188 et constaté qu'elle est bonne et nettement supérieure à celle des extraits obtenus à partir des espèces de Bacillus précédemment étudiées. On a, de plus, observé que cette stabilité est nettement améliorée par la présence de glucose à une concentration entre 0,1 et 1 M. D'autres sucres et polyols tels que le saccharose, le fructose, le mannitol ou le glycérol ont un effet stabilisant très inférieur. Enfin, on a constaté que la stabilité de l'enzyme est bonne à un pH compris entre 6 et 7, mais moins bonne à pH 7,5. A des pH plus alcalins tels que pH 8 et au delà la stabilité est mauvaise. Les conditions de stabilité qui viennent d'être décrites sont avantageusement utilisées pour la synthèse de la L-carnitine.

La présente invention ne se limite pas à l'utilisation d'extraits enzymatiques bruts de glucose déshydrogénase car on peut également, si on le désire, utiliser pour cette synthèse de la L-carnitine, des extraits purifiés de glucose déshydrogénase, obtenus à partir des extraits bruts qui viennent d'être décrits par utilisation des méthodes connues de purification des enzymes, telles que précipitation sélective avec le sulfate d'ammonium, chromatographie de divers types (échange d'ions, perméation sur gel, affinité) etc.

Exemple 1

On a cultivé une souche de Bacillus megaterium (souche IFP 180) sur un milieu contenant par litre 2,5 g de glucose, 2,5 g de phosphate bipotassique, 5 g de chlorure de sodium, 17 g d'hydrolysat tryptique de caséine et 3 g de peptone papaïnique de soja et ajusté à pH 7,0 après stérilisation 30 minutes à 115°C. Ces conditions sont telles qu'elles ne peuvent entraîner une limitation en phosphate de la culture effectuée.

Après 24 h de culture à 30°C les cellules ont été récoltées, suspendues dans un tampon phosphate de potassium 100 mM pH 7,5 puis un extrait enzymatique a été préparé par traitement aux ultra-sons (10 fois 15 sec) à froid (0 à 10°C) des suspensions cellulaires, suivi d'une centrifugation pour éliminer les débris cellulaires. L'activité enzymatique de la glucose déshydrogénase a été mesurée dans l'extrait ainsi obtenu et trouvée égale à 0,085 unité par mg de protéines de l'extrait. Une unité enzymatique (u) est défini comme la quantité d'enzyme permettant la transformation d'une micromole de glucose par minute dans les conditions de l'essai.

A partir de la souche IFP 180, des mutants de sporulation naturels ont été sélectionnés par repiquages successifs sur le milieu qui vient d'être décrit. Après cinq repiquages, on a constaté que l'activité glucose déshydrogénase des cultures était améliorée. On a poursuivi les repiquages jusqu'à vingt et obtenu des colonies isolées par étalement sur milieu solide des différentes cultures successives effectuées. Ces colonies ont ensuite été cultivées et traitées dans les mêmes conditions que la souche initiale IFP 180 et les activités enzymatiques de la glucose déshydrogénase ont été mesurées dans les extraits obtenus. La meilleure activité enzymatique, égale à 0,50 u par mg de protéines, a été obtenue à partir d'une culture de l'une de ces colonie désignée sous le nom de souche IFP 188. On a de plus constaté que la souche IFP 188 a un taux de sporulation très faible (inférieur à 0,4%) et constitue donc un mutant de sporulation. Le mutant B. megaterium IFP 188 a été retenu pour la production de glucose déshydrogénase dans les essais ultérieurs.

La stabilité de la glucose déshydrogénase a été mesuré dans les extraits enzymatiques de cette souche IFP 188. Dans ces essais, les extraits ont été stérilisés par filtration et conservés à 30°C dans un tampon phosphate de potassium à pH 7,0. La mesure périodique des activités glucose déshydrogénase des extraits conservés dans ces conditions a fourni des temps de demi-vie (temps requis pour la perte de la moitié de l'activité enzymatique) d'environ 15 heures.

L'addition de glucose 0,6 M augmente considérablement la stabilité de l'enzyme dans les extraits bruts puisque aucune perte d'activité n'a été observée en 21 jours dans les conditions ci-dessus. L'effet de stabilisation du glucose est dépendant de la concentration en glucose. A une concentration de 0,2 M le temps de demi-vie est de 40 à 50 h; il est d'environ 12 jours à 0,4 M.

A titre de comparaison, l'extrait de glucose déshydrogénase obtenu avec Bacillus subtilis n'avait qu'une activité de 0,045 unité/mg de protéines de l'extrait.

L'activité des meilleurs mutants de Bacillus subtilis n'a pas dépassé 0,085 u/mg de protéines. La souche sauvage de Bacillus subtilis et ses mutants avaient tous des temps de demi-vie compris entre 2 et 6 heures.

Exemple 2

On a cultivé la souche de Bacillus megaterium IFP 188 dans un fermenteur de 20 renfermant un milieu contenant 30 g/l de peptone papaïnique de soja. La stérilisation initiale était faite comme dans l'exemple 1. Le pH était maintenu à 7,3 par addition d'ammoniaque 10 N. Le milieu était aéré par injection de 0,5 l d'air par 1 de milieu par minute. Après 20 h les cellules étaient récoltées et des extraits enzymatiques préparés comme l'exemple 1. Une activité glucose déshydrogénase de 0,002 u par mg de protéine a été mesurée dans de tels extraits.

Le même souche a été cultivée dans les mêmes conditions sauf que le milieu contenait 10 g/l de glucose en plus de la peptone papaïnique de soja. Les extraits enzymatiques préparés comme précédemment avec les cellules bactériennes obtenues dans cette culture avaient une activité glucose déshydrogénase de 0.42 u par mg de protéines.

Exemple 3

Une autre culture de la souche IFP 188 a été effectuée comme dans l'exemple 2 sauf que le milieu de culture d'un volume de 16 l était constitué par de l'eau de ville dans laquelle on injectait tout au long de la culture une solution contenant 100 g/kg de liqueur de trempe de maïs («corn steep liquor») et 400 g/kg de glucose. La vitesse d'injection de ce mélange était de 50 ml/heure. Les extraits enzymatiques préparés avec les cellules obtenue récoltées après 20 h de croissance avaient une activité glucose déshydrogénase égale à 0,60 u par mg de protéines.

Exemple 4

Les extraits enzymatiques de glucose déshydrogénase préparés dans l'exemple 3 ont été utilisés pour effectuer la synthèse de la L-carnitine dans les conditons suivantes: on a utilisé un réacteur de 2 l thermostaté à 25°C et contenant 500 ml d'un milieu contenant les composants suivants aux concentrations indiquées en millimoles par litre (mM): phophate d'ammonium 50 mM pH 7,0, NAD$^+$ 1 mM, glucose 600 mM, carnitine déshydrogénase (préparée comme décrit dans l'exemple 1 du brevet français N° 2 398 046) 450 unités (soit 10 ml d'extrait enzymatique) et glucose déshydrogénase 150 unités (soit 10 ml d'extrait enzymatique brut). On injectait alors une solution contenant 800 millimoles par litre de bromhydrate de déshydrocarnitine (amenée à pH par addition d'acide chlorhydrique concentré). La vitesse d'injection était de 5 ml/h. Le pH était maintenu à 7,0 par addition d'ammoniaque 2 N, contrôlée au moyen d'un pH-mètre-titrateur automatique. La quantité de L-carnitine formée, dosée par méthode enzymatique était après 48 heures de 30,5 g.

Revendications

1. Procédé de production de glucose déshydrogénase consistant à cultiver une souche de Bacillus dans un milieu de culture et à extraire l'enzyme obtenu, caractérisé en ce que la souche de Bacillus est le mutant IFP 188 (ATCC 39.118) et la culture est effectuée en présence de glucose à une concentration comprise entre 1 et 40 g/l.

2. Procédé selon la revendication 1, dans lequel la culture est effectuée dans un milieu de culture qui renferme à la fois au moins 1 g/l de glucose et au moins 1 g/l de liqueur de trempe de maïs.

3. Procédé selon l'une des revendications 1 à 2, dans lequel le glucose a été introduit progressivement au cours de la culture.

## Patentansprüche

1. Verfahren zur Herstellung von Glucose-Dehydrogenase, wobei man einen Stamm-Bacillus in einem Kulturmilieu kultiviert und das erhaltene Enzym extrahiert, dadurch gekennzeichnet, dass man als Bacillus-Stamm die Mutante IFP 188 (ATCC 39.118) verwendet und die Kultur in Gegenwart von Glucose bei einer Konzentration von 1 bis 40 g/l durchführt.

2. Verfahren gemäss Anspruch 1, dadurch gekennzeichnet, dass die Kultur in einem Kulturmilieu durchgeführt wird, welches gleichzeitig mindestens 1 g/l Glucose und mindestens 1 g/l Saft von Mais-Maische enthält.

3. Verfahren gemäss Ansprüchen 1 bis 2, dadurch gekennzeichnet, dass die Glucose progressiv während der Kultur eingeführt wird.

## Claims

1. A process for producing glucose dehydrogenase, consisting of cultivating a Bacillus strain in a culture medium and extracting the resultant enzyme, characterized in that the Bacillus strain is the mutant IFP 188 (ATC 39 118) and the culture is effected in the presence of glucose at a concentration of from 1 to 40 g/l.

2. A process according to claim 1, wherein the culture is effected in a culture medium which both comprises at least 1 g/l of glucose and at least 1 g/l of corn steep liquor.

3. A process according to one of claims 1 to 2, wherein glucose has been introduced progressively in the course of the culture.